# EUROPÄISCHE PATENTSCHRIFT

(11) **EP 3 713 522 B1**
(45) Veröffentlichungstag und Bekanntmachung des Hinweises auf die Patenterteilung: **06.12.2023**
(21) Anmeldenummer: 18785948.3
(22) Anmeldetag: 11.10.2018
(51) Int. Cl.: A61F 2/64

(54) **BREMSGELENK**
ARTICULATION WITH BRAKE
ARTICULATION AVEC FREIN

(30) Priorität: 24.11.2017 DE 102017127860
(43) Veröffentlichungstag der Anmeldung: 30.09.2020
(73) Patentinhaber: Ottobock SE & Co. KGaA, 37115 Duderstadt (DE)
(72) Erfinder: BOITEN, Herman, 37085 Göttingen (DE); FRICK, Eugen, 21031 Hamburg (DE)
(74) Vertreter: Gramm, Lins & Partner Patent- und Rechtsanwälte PartGmbB
(86) Internationale Anmeldenummer: PCT/EP2018/077739
(87) Internationale Veröffentlichungsnummer: WO 2019/101427

(56) Entgegenhaltungen:
- EP-A2- 2 502 607
- WO-A1-2016/198808
- US-B1- 6 960 175
- US-B1- 7 087 091

## Beschreibung

Die Erfindung betrifft ein Bremsgelenk mit einem Oberteil, einem um eine Schwenkachse schwenkbar daran gelagerten Unterteil und einer Bremseinrichtung, die zumindest ein Bremselement aufweist, das über einen Auslösemechanismus aus einem aktivierten, einer Flexionsbewegung zumindest bremsenden Zustand in einen deaktivierten, eine Flexionsbewegung zulassenden Zustand bringbar ist, wobei die Bremseinrichtung eine Extensionsbewegung nicht sperrt. Das Bremsgelenk ist insbesondere als ein Bremskniegelenk vorteilhaft einsetzbar, ist jedoch nicht auf diese Anwendung beschränkt. Das Bremsgelenk kann sowohl als Prothesenbremsgelenk als auch als Orthesenbremsgelenk, insbesondere als Bremskniegelenk eingesetzt werden. Neben einer Anwendung an unteren Extremitäten kann das Bremsgelenk auch an oberen Extremitäten eingesetzt werden. Insbesondere kann das Bremsgelenk bei Exoskeletten oder anderen Körperunterstützungssystemen eingesetzt werden. Auch andere, nicht prothetische oder orthetische Lösungen sind denkbar, wenn es notwendig ist, eine Drehbewegung in einer Richtung zu erlauben und in der anderen Richtung wahlweise zu erlauben oder zu verhindern bzw. zu behindern.

Bremsgelenke werden überwiegend als Bremskniegelenke eingesetzt, mit denen es möglich ist, während einer Axialbelastung des Unterschenkels eine Bremskraft auszuüben, um eine Flexion des Prothesenkniegelenkes, also ein Einbeugen des Oberteils relativ zu dem Unterteil, während der Standphase zu bremsen oder zu verhindern. Üblicherweise sind bei solchen Gelenken die Bremseinrichtungen oder Bremselemente standardmäßig geöffnet, so dass eine Verschwenkung jederzeit in beiden Richtungen möglich ist. Erst bei Aufbringen einer ausreichenden Axialkraft oder Vertikallast wird die Bremseinrichtung aktiviert und eine Verschwenkung in Flexionsrichtung gesperrt. Eine solche Situation tritt üblicherweise dann auf, wenn der Nutzer mit einem im Wesentlichen gestreckten Bein auftritt oder steht. Ein Bremskniegelenk ist aus der EP 0 353 336 B1 bekannt, in der ein künstliches Kniegelenk mit einem einen Zapfen zur Befestigung an einem Oberschenkelschaft aufweisenden Klemmkörper beschrieben ist, der gegen einen in einem Kniegelenkgehäuse befindlichen Bremskörper schwenkbar und an ihm bei vertikaler Kniegelenkbelastung die Fortführung der Schwenkbewegung sperrend anlegbar ist.

Aus der EP 1 838 253 B1 ist ein künstliches Kniegelenk mit einer Einrichtung zur Standphasenkontrolle bekannt, das ein Verriegelungselement aufweist, um das Kniegelenk in einem geraden Zustand zu halten. Mit einer weiteren Einrichtung wird der Zustand des Verriegelungselementes bestimmt, wobei eine Kontrollachse derart positioniert ist, dass das Verriegelungselement aktiviert wird, wenn eine Belastung auf das im Einsatz befindliche Gelenk durch eine Linie hinter oder posterior der Kontrollachse verläuft. Eine Deaktivierung findet statt, wenn die Belastung durch eine Linie vor der oder anterior der Kontrollachse verläuft. Bei einer Vorfußbelastung wird über die Steuerachse somit die Sperre gelöst, bei vollständiger Streckung rastet die Sperre wieder ein.

Die US 8 915 969 B2 betrifft eine Prothese mit zwei künstlichen Gliedmaßen, die über ein Gelenk miteinander verbunden sind. An den beiden Gliedmaßen ist ein Hy-draulikdämpfer angeordnet, der einer Relativbewegung der beiden künstlichen Gliedmaßen um das Gelenk einen Widerstand entgegensetzt. Der Dämpfer wird bei einer Vorfußlast freigeschaltet und nutzt die Hydraulikströmung als ein Signal für ein Ventil, in der Schwungphase geöffnet zu bleiben.

Die WO 2015/126792 A1 betrifft ein Prothesenkniegelenk mit einem Oberteil und einem verschwenkbar daran gelagerten Unterteil mit einer Bremseinrichtung, die schwenkbar an einem Gehäuse an einem ersten Befestigungspunkt und an einem Chassis an einem zweiten Befestigungspunkt gelagert ist. Eine Extensionsunterstützung ist in dem Prothesenkniegelenk angeordnet und weist einen Vorspannmechanismus auf.

Problematisch bei Bremsgelenken, insbesondere bei mechanischen Bremskniegelenken, kann der Umstand sein, dass eine Flexion nur dann verhindert wird, wenn eine Vertikallast aufgebracht wird. In einer Stolpersituation kann dies dazu führen, dass das Prothesenkniegelenk unerwünscht einbeugt, da keine oder eine zu geringe Vertikallast aufgebracht wird. Auch ein entspanntes Auftreten ohne besondere Beachtung der Prothesenlast ist mit den meisten Prothesenkniegelenken gemäß dem Stand der Technik nicht oder nur eingeschränkt möglich, da eine mehr oder weniger gestreckte Stellung des Prothesenkniegelenkes notwendig ist, um eine Bremswirkung oder eine Verriegelungswirkung zu erzielen.

Die EP 2 502 607 A2 beschreibt eine Gelenkeinrichtung für Orthesen oder Prothesen, insbesondere eine Kniegelenkseinrichtung. Die Gelenkeinrichtung weist an einem Unterteil eine Kippplatte auf, die bei einer Fersenlast ein federvorgespanntes Seil, das um einen Ring geschlungen ist, spannt. An dem Ring sind Stifte befestigt, über die bei einer Verlagerung des Ringes Rollkörper aus einem Eingriff mit einer Bremstrommel eines Freilaufes gebracht werden.

Aufgabe der vorliegenden Erfindung ist es, ein Bremsgelenk bereitzustellen, das ein erhöhtes Maß an Sicherheit und Komfort für den Prothesennutzer bereitstellt.

Erfindungsgemäß wird diese Aufgabe durch ein Bremsgelenk mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Das Bremsgelenk mit einem Oberteil, einem um eine Schwenkachse daran gelagerten Unterteil und einer Bremseinrichtung, die zumindest ein Bremselement aufweist, das über einen Auslösemechanismus aus einem aktivierten, eine Flexionsbewegung zumindest bremsenden Zustand in einen deaktivierten, eine Flexionsbewegung zulassenden Zustand bringbar ist, wobei die Bremseinrichtung eine Extensionsbewegung nicht sperrt, sieht vor, dass der Bremseinrichtung ein Aktuator zugeordnet ist, der das zumindest eine Bremselement während der Flexionsbewegung in dem deaktivierten Zustand hält. Über den Aktuator ist es möglich, während der gesamten Flexionsbewegung eine Kraft bereitzustellen, die die Bremseinrichtung aus einer aktivierten in einen deaktivierten Zustand bewegt und dort hält. Sobald die Bewegungsrichtung umkehrt, also von einer Flexionsbewegung in eine Extensionsbewegung übergeht, fällt die Kraft durch den Aktuator weg, so dass das Bremselement nicht mehr in dem deaktivierten Zustand gehalten wird.

Der Auslösemechanismus ermöglicht zunächst grundsätzlich, dass die Bremseinrichtung aus einem aktivierten Zustand, beispielsweise während der Standphase, überhaupt in einen Zustand gebracht wird, in der eine Flexionsbewegung möglich ist. Dies kann beispielsweise über einen Mechanismus erfolgen, der eine Vorfußlast detektiert. Die Bremseinrichtung wird nach der Detektion einer Vorfußlast deaktiviert. Nach der Einleitung einer Flexionsbewegung wird das Bremselement durch den Aktuator außer Eingriff mit der übrigen Bremseinrichtung gehalten. Sollte nach dem Auslösen durch den Auslösemechanismus und einer Deaktivierung der Bremseinrichtung keine Flexionsbewegung stattfinden, ist vorteilhafterweise vorgesehen, dass das Bremselement wieder in Eingriff mit den übrigen Komponenten der Bremseinrichtung gebracht wird, um das Gelenk wieder zu sperren. Der Auslösemechanismus weist einen Auslösehebel auf, der mit dem zumindest einen Bremselement gekoppelt ist. Die Kopplung kann über das Betätigungselement erfolgen, so dass bei einer entsprechenden Betätigung des Auslösehebels das Bremselement aus einer aktivierten Stellung in eine deaktivierte Stellung gebracht werden kann. Der Auslösehebel kann beispielsweise durch einen Schalter, einen Hebel oder durch einen Kabelzug manuell betätigt werden, um willentlich das Bremsgelenk zu entsperren. Bei der Ausgestaltung des Bremsgelenkes als Bremskniegelenk kann dadurch beispielsweise das Hinsetzen erleichtert werden.

Der Aktuator kann das zumindest eine Bremselement während der Extensionsbewegung in den aktivierten Zustand bringen, also eine aktive Verlagerung des Bremselementes in denjenigen Zustand bewirken, in dem eine Bremswirkung in Flexionsrichtung erfolgt, eine Extensionsbewegung jedoch weiter möglich ist. Der Aktuator hält dann nicht nur das Bremselement in einem deaktivierten Zustand, nachdem das Bremselement durch den Auslösemechanismus aus einem sperrenden Zustand in einen freigegebenen Zustand gebracht worden ist, sondern bewegt das Bremselement aktiv wieder in den aktivierten Zustand, beispielsweise indem das Bremselement an eine Bremsscheibe oder eine Bremstrommel angelegt wird. Erfolgt nach einer ausgeführten Extensionsbewegung wieder eine Flexionsbewegung, beispielsweise wenn das Unterteil an einen Gegenstand stößt, verriegelt die Bremsbewegung das Gelenk über die Bremseinrichtung, so dass durch die nicht vorhandene Flexionsbewegung der Aktuator das Bremselement nach einer Bewegungsumkehr nicht mehr in den deaktivierten Zustand bringen kann.

Alternativ oder ergänzend zu einer aktiven Verstellung des Bremselementes durch den Aktuator ist in einem Ausführungsbeispiel dem zumindest einen Bremselement ein Rückstellelement zugeordnet, das das zumindest eine Bremselement in Richtung auf den aktivierten Zustand belastet oder bewegt. Das Rückstellelement kann als eine mechanische Feder oder pneumatische Feder ausgebildet sein. Innerhalb des Rückstellelementes kann ein Verzögerungsglied angeordnet sein, das eine unmittelbare Rückstellung des Bremselementes nach Wegfall einer Deaktivierungskraft durch den Auslösemechanismus verhindert. Dadurch ist es möglich, eine gewisse Zeitspanne einzustellen, innerhalb derer nach Deaktivierung durch den Auslösemechanismus das Bremsgelenk eine Flexionsbewegung ausführen kann. Das Gelenk wird somit über einen gewissen Zeitraum beweglich gehalten. Tritt dann eine Flexionsbewegung auf, wird für die weitere Flexionsbewegung durch den Aktuator eine Kraft bereitgestellt, die der Kraft des Rückstellelementes entgegenwirkt. Findet dann eine Umkehr der Bewegungsrichtung von Flexion zu Extension statt, wird entweder das Bremselement alleine durch das Rückstellelement in den aktivierten Zustand bewegt oder in Verbindung mit einer Bewegung durch den Aktuator.

Das zumindest eine Bremselement kann in Flexionsrichtung selbstverstärkend und in Extensionsrichtung deaktivierend gelagert sein. Dies kann beispielsweise durch eine Lagerung ähnlich einem Freilauf erfolgen, bei der in Flexionsrichtung das Bremselement an einer Bremsfläche anliegt und durch die Reibkraft zwischen dem Bremselement und der Bremsfläche das Bremselement weiter gegen die Bremsfläche gedrückt wird. Umgekehrt wird in Extensionsrichtung das Bremselement mit einer Kraft beaufschlagt, die keine Selbstverstärkung zulässt, so dass das Bremselement auch ohne eine Aktuatorwirkung nur gegebenenfalls über das Rückstellelement an die Bremsfläche angedrückt wird. Dieses Andrücken bewirkt zwar eine geringfügige Abbremsung der Bewegung, jedoch nicht ein Sperren des Bremsgelenkes in Extensionsrichtung.

Das zumindest eine Bremselement ist in einer Ausgestaltung in einer Bremstrommel schwenkbar an einem Träger gelagert. Die Lagerung ist so gewählt, dass der Schwenkpunkt des Bremselementes radial außerhalb der Schwenkachse liegt. Die Berührfläche des Bremselementes mit der Bremstrommel ist so in Umfangsrichtung von der Verbindungslinie zwischen der Schwenkachse und dem Lagerungspunkt des Bremselementes beabstandet, dass sie dem Lagerungspunkt vorläuft und bei einer Flexionsbewegung das Bremselement gegen die Bremstrommel gedrückt wird. Das Bremselement kann gebogen ausgeführt sein, wodurch das zumindest eine Bremselement in der Extensionsbewegung von der Bremsfläche weggedrückt und in der Flexionsbewegung gegen die Bremsfläche gedrückt wird.

In einer Ausgestaltung der Erfindung ist ein Betätigungselement verdrehbar zu dem Träger gelagert und mit dem Auslösemechanismus gekoppelt. Insbesondere bei mehreren Bremselementen wird über das Betätigungselement eine synchronisierte Bewegung aller Bremselemente erreicht. Das Betätigungselement als Verbindungsring für die Bremselemente dienen, die an dem Träger gelagert sind, so dass durch eine einmalige Ankopplung des Auslösemechanismusses an das Betätigungselement sämtliche Bremselemente in der gleichen Richtung verschwenkt werden können.

In einer Ausgestaltung der Erfindung ist das Unterteil in zumindest zwei Komponenten unterteilt, die um eine Steuerachse verschwenkbar aneinander gelagert sind. Bei einer Auslösebelastung, bei einem Bremskniegelenk, beispielsweise eine Vorfußlast, wird eine Verschwenkbewegung der Unterteilkomponenten zueinander bewirkt, durch die der Auslösemechanismus das Bremselement entweder unmittelbar oder über das Betätigungselement deaktiviert. Bei mehreren Bremselementen kann der Auslösemechanismus entweder direkt alle Bremselemente bewegen oder die Bewegung über das Betätigungselement erfolgen.

Das Betätigungselement ist bevorzugt auch mit dem Aktuator gekoppelt, so dass alle Bremselemente während der Flexionsbewegung durch den Aktuator außer Eingriff mit der Bremsfläche gebracht oder gehalten werden.

In einer Ausgestaltung der Erfindung ist der Aktuator als Rotationsdämpfer ausgebildet, der die Flexionsbewegung in eine den deaktivierten Zustand beibehaltene Kraft umwandelt. Die Dämpfung kann als Flüssigkeitsdämpfung oder auch als Festkörperreibung erzeugt werden.

In einer Ausgestaltung ist der Aktuator über einen Mitnehmer verschwenkbar um die Schwenkachse mit dem Oberteil oder Unterteil gekoppelt, wodurch die Relativbewegung zwischen dem Oberteil und dem Unterteil auf das zumindest eine Bremselement überträgt. Der Mitnehmer kann unmittelbar auf das Bremselement oder die Bremselemente einwirken, ebenso kann der Mitnehmer über das Betätigungselement auf die Bremselemente einwirken.

Der Aktuator kann über eine Verzahnung mit dem Oberteil oder dem Unterteil gekoppelt sein, so dass ein zuverlässiger mechanischer Antrieb über eine formschlüssige Kraftübertragung bei einer Relativbewegung zwischen dem Oberteil und dem Unterteil auf den Aktuator erfolgen kann. Statt einer Verzahnung kann auch über eine Reibradkopplung eine Kraftübertragung erreicht werden.

Eine Variante der Erfindung sieht vor, dass der Aktuator als sensorgesteuerter Antrieb ausgebildet ist, so dass statt einer rein mechanischen Steuerung des Bremsgelenkes eine sensorbasierte, elektronische Lösung für den Aktuator zum Einsatz kommen kann. Die Ausgestaltung der Bremseinrichtung als rein mechanisch wirkende Bremse kann dabei beibehalten werden, lediglich die Sensierung und Erfassung einer Bewegungsumkehr und eine Abschaltung eines deaktivierenden Antriebs oder eines deaktivierenden Elementes, beispielsweise die Abschaltung eines Solenoids oder einer Magnetspule, die die Bremselemente oder das Bremselement außer Eingriff mit der Bremsfläche hält, verändert sich zu dem oben beschriebenen Aufbau. Auch hier kann der Aktuator das zumindest eine Bremselement in den aktivierten Zustand bewegen, sei es durch eine unmittelbare Verlagerung des Bremselementes in Richtung auf die Bremsfläche, durch eine Verlagerung des Betätigungselementes zur Aktivierung sämtlicher Bremselemente oder durch Unterstützung einer Rückstellkraft durch ein Rückstellelement, das als Feder ausgebildet sein kann.

Bevorzugt ist das Bremsgelenk als Bremskniegelenk ausgebildet. Alternativ zu einer Ausgestaltung als Bremskniegelenk kann das Bremsgelenk als jegliches Gelenk ausgebildet sein, das in einer Richtung frei beweglich und in der anderen wahlweise sperrbar ist. Statt einer Extensionsbewegung kann auch eine Flexionsbewegung stets möglich sein und eine Sperrung in Extensionsbewegung erfolgen. Die Ausführungen hinsichtlich der Extensions- und Flexionsbewegung gelten auch entsprechend umgekehrt.

Nachfolgend wird ein Ausführungsbeispiel der Erfindung anhand der beigefügten Figuren näher erläutert. Es zeigen:
- Figur 1 -: ein Bremsgelenk als Bremskniegelenk in einer teildemontierten Seitenansicht;
- Figur 2 -: das Gelenk in einer Seitenansicht;
- Figur 3 -: eine vergrößerte Darstellung der Bremselemente in einer Bremstrommel;
- Figur 4 -: eine vergrößerte Darstellung einer geschlossenen Bremse;
- Figur 5 -: eine vergrößerte Darstellung einer geöffneten Bremse;
- Figur 6 -: eine Darstellung eines gebeugten Gelenkes mit geöffneter Bremse;
- Figur 7 -: eine Darstellung des Gelenkes bei Bewegungsumkehr;
- Figur 8 -: eine Detaildarstellung mit Aktuator und Verzahnung;
- Figur 9 -: eine Variante der Figur 8 im gebeugten Zustand;
- Figur 10 -: eine perspektivische Einzeldarstellung der Bremselemente auf einem Träger mit Betätigungselement und Aktuator; sowie
- Figur 11 -: die Darstellung gemäß Figur 10 in eingebautem Zustand.

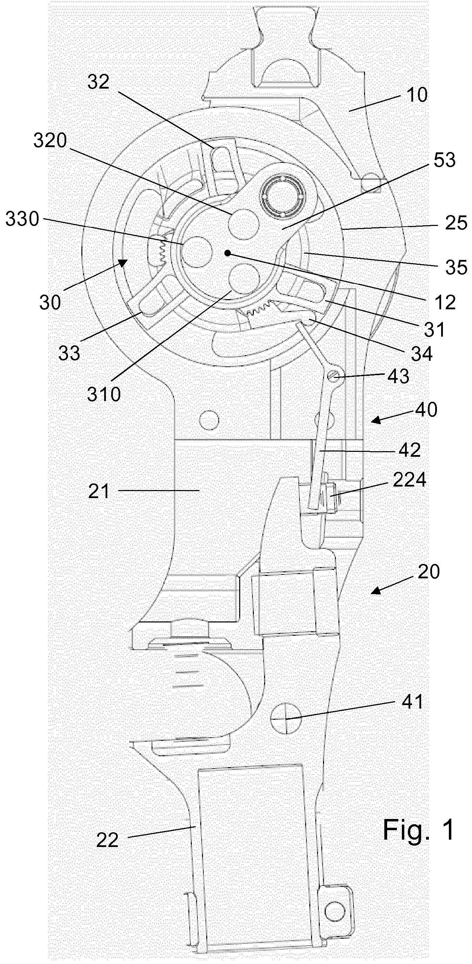

In der Figur 1 ist in einer teilweise freigeschnittenen Darstellung ein Gelenk in Gestalt eines Prothesenkniegelenkes dargestellt. Das Prothesengelenk weist ein Oberteil 10 und ein um eine Schwenkachse 12 daran gelagertes Unterteil 20 auf. Statt eines dargestellten einachsigen Gelenkes kann das Prothesengelenk auch als polyzentrisches Gelenk ausgebildet sein. Das Unterteil 20 weist zwei Komponenten 21, 22 auf, die verschwenkbar über eine Steuerachse 41 aneinander gelagert sind. Die dem Oberteil 10 zugewandte Unterteilkomponente 21 bildet zudem eine Bremstrommel 25 aus, in der eine Bremseinrichtung 30 mit drei Bremselementen 31, 32, 33 gelagert sind. Die Bremseinrichtung 30 ist drehfest mit dem Oberteil 10 verbunden.

Weiterhin ist an der proximalen Unterteilkomponente 21 ein Auslösemechanismus 40 angeordnet, der einen Auslösehebel 42 aufweist, der um eine Schwenkachse 43 an dem Unterteil 20 gelagert ist. Auf den Auslösehebel 42 wirkt bei einer Vorfußlast oder einer frontal, in der zeichnerischen Darstellung rechts zu der Steuerachse 41 verlaufenden Kraft eine Verschwenkung der proximalen Unterteilkomponente 21 um die Schwenkachse 41 in Uhrzeigerrichtung. An der distalen Unterteilkomponente 22 ist eine Aufnahme 224 für den Auslösehebel 42 angeordnet, der den unteren Teil des Auslösehebels 42 nach links bewegt, so dass eine Verschwenkung des Auslösehebels 42 in Uhrzeigersinn um die Schwenkachse 43 ausgeführt wird. Dadurch wird der obere Schenkel des zweiarmigen Auslösehebels 42 gegen eine Lasche 34 gedrückt, die Teil eines Betätigungselementes 35 ist. Dieses Betätigungselement 35 ist ein Verbindungsring, der die drei Bremselemente 31, 32, 33, die auf einem später erläuterten Träger verschwenkbar gelagert sind, gleichzeitig aktuiert werden können. Das Betätigungselement 35 ist über Verzahnungen mit den Bremselementen 31, 32, 33 gekoppelt und wird durch den Auslösehebel 42 entgegen dem Uhrzeigersinn verdreht. Die Innenverzahnung auf dem Betätigungselement 35 greift in Außenverzahnungen an den Bremselementen 31, 32, 33 ein und führt dazu, dass die Bremselemente, die schwenkbar um Zapfen 310, 320, 330 gelagert sind, ebenfalls verschwenkt werden und jeweils eine Verschwenkung entgegen dem Uhrzeigersinn um die Zapfen 310, 320, 330 ausführen. Dadurch werden die Bremselemente 31, 32, 33 von der Innenseite der Bremstrommel 25 abgehoben, so dass insgesamt die Bremse gelöst ist und das Oberteil 10 relativ zu dem Unterteil 20 verschwenkbar ist.

Ebenfalls drehfest mit dem Oberteil 10 ist ein Mitnehmer 53 gelagert, der mit den Zapfen 310, 320 330 gekoppelt ist und als Aufnahme für einen Aktuator 50 dient, der später erläutert werden wird.

In dem dargestellten, gelösten Zustand ist es möglich, das Oberteil 10 entgegen dem Uhrzeigersinn um die Schwenkachse 12 zu verlagern, um eine Flexion des Gelenkes zu bewirken. Über den Aktuator wird eine Kraft auf die Bremselemente 31, 32, 33 ausgeübt, die diese von der Bremsfläche der Bremstrommel 25 abheben. Die Bremselemente 31, 32, 33 sind jeweils versetzt zu der Linie zwischen der Schwenkachse 12 und den Lagerungszapfen 310, 320 330 orientiert ausgebildet, so dass die Kontaktflächen der Bremselemente 31, 32, 33 mit der Bremstrommel 25 bei einer Extensionsbewegung lediglich auf der Oberfläche der Bremstrommel 25 entlanggleiten. Wird eine Flexionsbewegung ausgeübt, also eine Verschwenkung des Oberteils 10 entgegen dem Uhrzeigersinn, legen sich die Kontaktflächen der Bremselemente 31, 32, 33 an die Bremstrommel 25 und verkeilen sich dort, so dass es zu einer Blockade der Verschwenkbewegung in Flexionsrichtung kommt. Eine Extensionsbewegung ist weiterhin möglich, eine Flexionsbewegung wird vollständig und selbstverstärkend gesperrt.

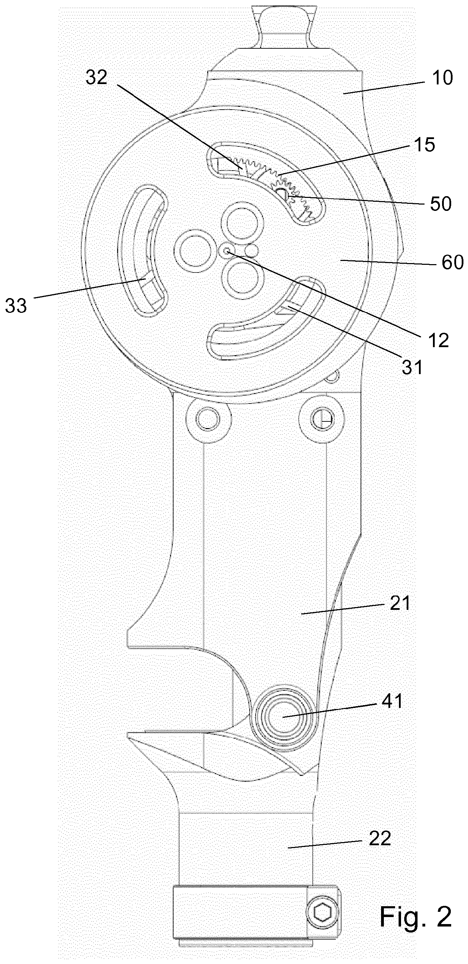

Figur 2 zeigt das Gelenk gemäß Figur 1 in seitlicher Darstellung mit dem daran montierten Träger 60, einer Verzahnung 15, die an dem Unterteil 20 axial versetzt zu den Bremselementen 31, 32, 33 in der Bremstrommel angeordnet ist. Innerhalb der Verzahnung 15 läuft ein Zahnrad des Aktuators 50 ab, wenn das Oberteil 10 um die Schwenkachse 12 verschwenkt wird. Der Aktuator 50 ist in dem dargestellten Ausführungsbeispiel als Rotationsdämpfer ausgebildet, der die Drehbewegung zwischen dem Zahnrad des Aktuators 50 und der Verzahnung 15, die als Innenverzahnung auf einem außenliegenden Ring ausgebildet ist, auf die Bremselemente 31, 32, 33 überträgt. In dem dargestellten Ausführungsbeispiel erfolgt die Übertragung dadurch, dass eine mit der ersten Verzahnung gekoppelte zweite Verzahnung in eine Außenverzahnung des Betätigungselementes 35 eingreift.

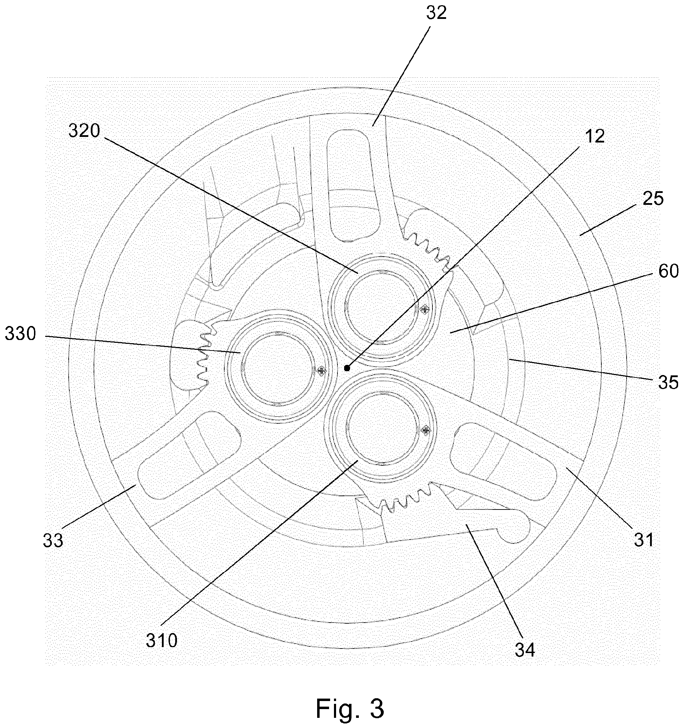

Figur 3 zeigt eine vergrößerte Darstellung der Bremseinrichtung mit der Bremstrommel 25, den drei Bremselementen 30, die verschwenkbar um die Zapfen 310, 320 330 auf dem Träger 60 gelagert sind und dem Betätigungselement 35, das um einen geringen Winkel um den Träger 60 drehbar ist. Das Betätigungselement 35 weist drei Innenverzahnungssegmente auf, von denen eines mit der Lasche 34 gekoppelt und über den Auslösehebel 42 betätigbar ist. In dem dargestellten Zustand liegen die Bremselemente 31, 32, 33 an der Bremsfläche der Bremstrommel 25 an und sperren eine Bewegung entgegen dem Uhrzeigersinn, so dass der Träger 60 nicht im Uhrzeigersinn verdreht werden kann. Eine im Uhrzeigersinn gerichtete Bewegung des Oberteils 10 ist jederzeit möglich, da aufgrund der von innen nach außen gesehen leicht nach links versetzten Ausrichtung der Kontaktflächen der jeweiligen Bremselemente 31, 32, 33 von der Schwenkachse 12 zu den jeweiligen Lagerzapfen 310, 320, 330 die Bremselemente 31, 32, 33 auf der Bremstrommel entlanggeschleppt werden.

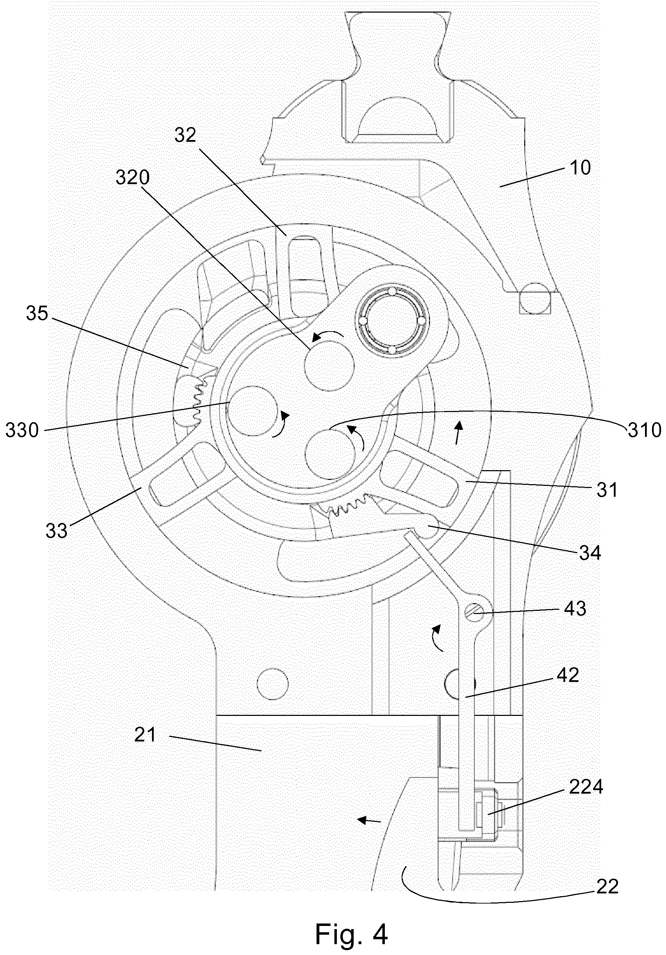

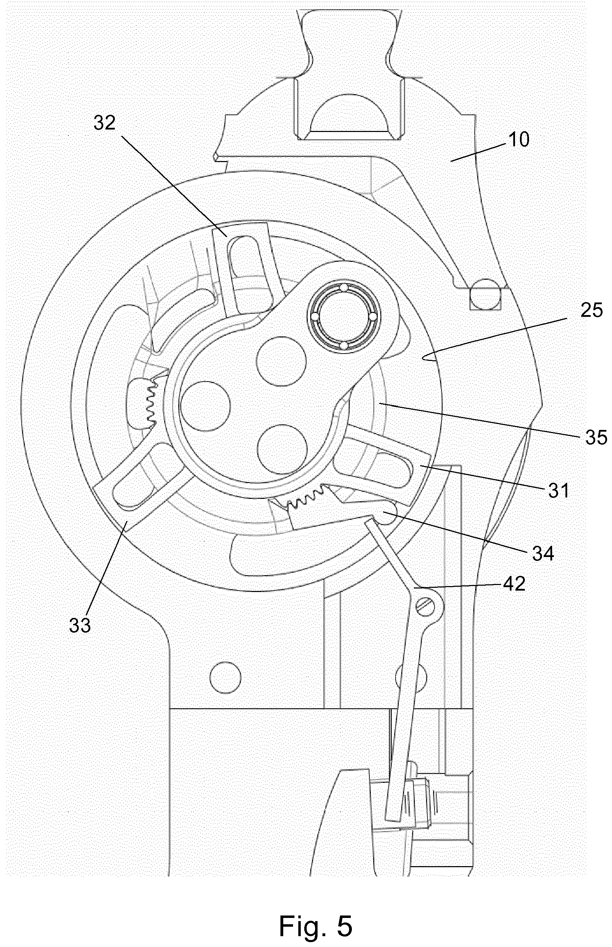

Figur 4 zeigt eine vergrößerte Darstellung des Bremsgelenkes mit einer geschlossenen Bremse. Die distale Unterteilkomponente 22 und die Aufnahme 224 befinden sich noch nicht im Eingriff mit dem Auslösehebel 42, die beiden Unterteilkomponenten 21, 22 sind noch gerade ausgerichtet und noch nicht um die Steuerachse 41 relativ zueinander verschwenkt. Der kurze Schenkel des Auslösehebels 42 befindet sich noch nicht im Eingriff mit dem Betätigungselement 35 über die Lasche 34. Wird nun die distale Unterteilkomponente 22 um die nicht dargestellte Steuerachse 41 entgegen dem Uhrzeigersinn verschwenkt, wird der lange Schenkel des Auslösehebels 42 von der Aufnahme 224 nach links bewegt, was zu einer Verschwenkung um die Schwenkachse 43 führt. Der kurze Hebel des Auslösehebels 42 wird dann im Uhrzeigersinn um die Schwenkachse 43 in Richtung auf die Lasche 34 verlagert, wodurch das Betätigungselement 35 um die Schwenkachse 42 entgegen dem Uhrzeigersinn verlagert wird. Dies führt zu einer Verschwenkung der Bremselemente 31, 32, 33 um die Lagerzapfen 310, 320 330 entgegen dem Uhrzeigersinn, wie dies durch die Pfeile dargestellt ist. Der sich dann einstellende Zustand ist in der Figur 5 dargestellt. Die jeweiligen Bremselemente 31, 32, 33 sind von der inneren Oberfläche der Bremstrommel 25 abgehoben, die Bremselemente 31, 32, 33 befinden sich in einem deaktivierten Zustand und das Oberteil kann entgegen dem Uhrzeigersinn verschwenkt werden.

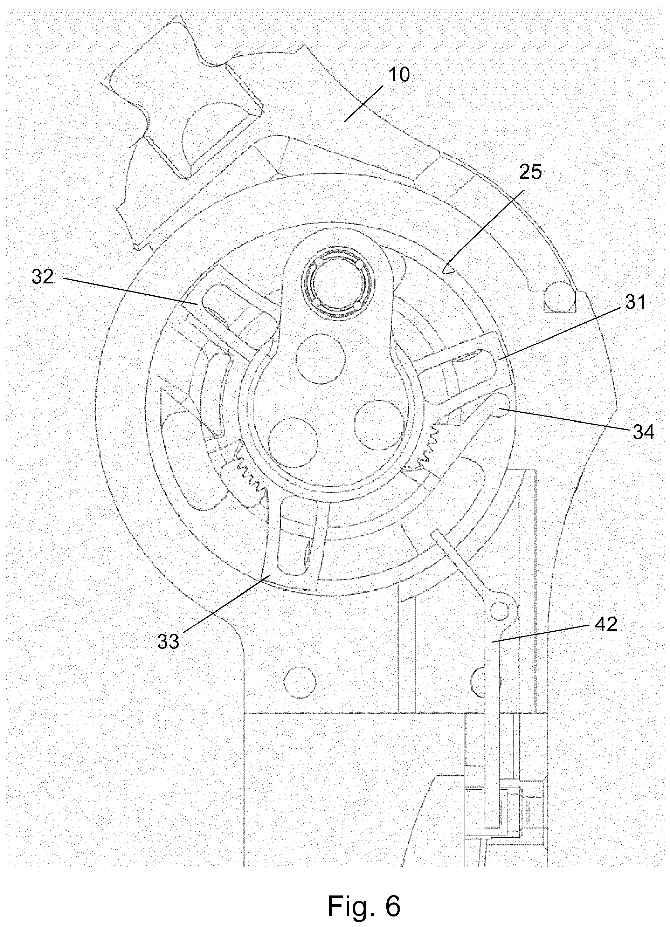

Die Verschwenkbewegung des Oberteils 10 relativ zu dem Unterteil 20 mit der geöffneten Bremse ist in der Figur 6 dargestellt. Die Lasche 34 befindet sich nicht mehr in Eingriff mit dem Auslösehebel 42, der in seiner Ausgangsstellung zurückgekehrt ist, nachdem die Auslösekraft, beispielsweise eine Vorfußlast, weggefallen ist. Dies kann beispielsweise bei der Ausgestaltung des Kniegelenkes als Bremskniegelenk während der Schwungphase der Fall sein. Während des Einbeugens wird eine permanente Kraft auf das Betätigungselement 35 oder den Verbindungsring ausgeübt, um die Bremselemente 31, 32, 33 in dem deaktivierten Zustand zu halten.

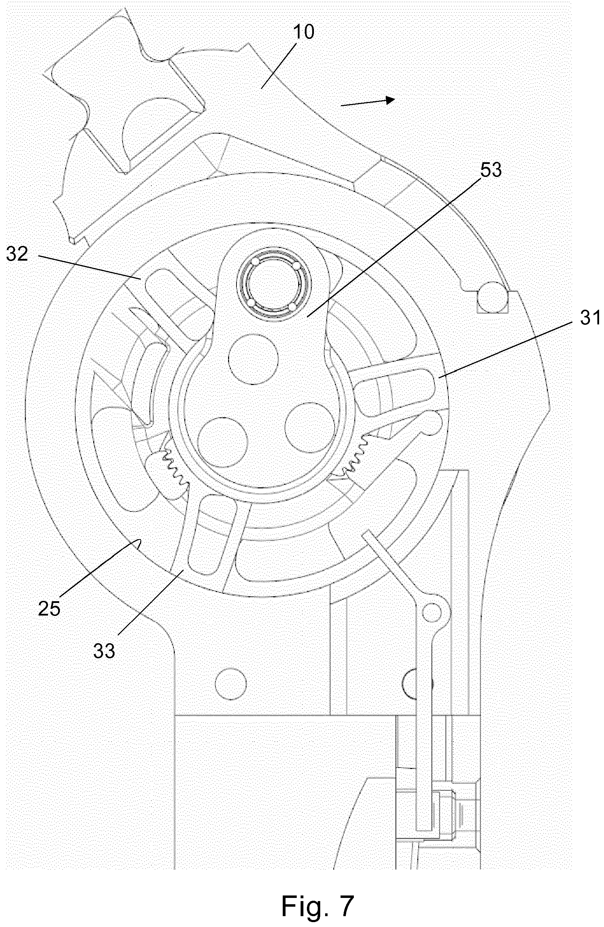

In der Figur 7 ist der Zustand der Bremseinrichtung 30 bei einer Bewegungsumkehr gezeigt, also wenn das Oberteil 10 in Uhrzeigersinn verschwenkt wird, was durch den Pfeil angedeutet wird. Die die Bremselemente 31, 32, 33 von der Bremstrommel abhebenden, in den deaktivierten Zustand bringenden Kräfte durch den Aktuator 50, der durch den Mitnehmer 30 bewegt wird, fallen weg. Die Bremselemente 31, 32, 33 liegen an der Bremstrommel 25 an und gleiten aufgrund ihrer nachlaufenden Orientierung auf der Bremsfläche entlang. Eine nachlaufende Orientierung bedeutet, dass die Kontaktflächen der Bremselemente 31, 32, 33 in der Verschwenkrichtung hinter der Verbindungslinie zwischen der Schwenkachse 12 und den Verschwenkachsen der Lagerzapfen 310, 320, 330 liegen. In Flexionsrichtung sind die Kontaktflächen vorlaufend angeordnet, so dass es hier zu einer selbstverstärkenden Anlage aufgrund der Tendenz zur Verschwenkung der Bremselemente 31, 32, 33 um die Lagerzapfen 310, 320 330 in Uhrzeigerrichtung kommt. Eine Extension ist somit weiterhin möglich, eine Flexion wird sofort durch die Bremselemente 31, 32, 33 gesperrt. Sollte also bei einem Bremskniegelenk nach Beendigung der Flexion eines minimalen Kniewinkels nach einer Bewegungsumkehr und einer Zunahme des Kniewinkels eine Störung in dem Bewegungsablauf auftreten, beispielsweise wenn ein Prothesenfuß an einem Hindernis hängenbleibt und der Patient stolpert, wird das Gelenk sofort verriegelt und eine Verschwenkbewegung in Flexionsrichtung kann nicht mehr stattfinden. Das Gelenk ist sicher, auch ohne dass Kräfte in Längserstreckung des Unterteils 20 auf das Gelenk wirken müssten.

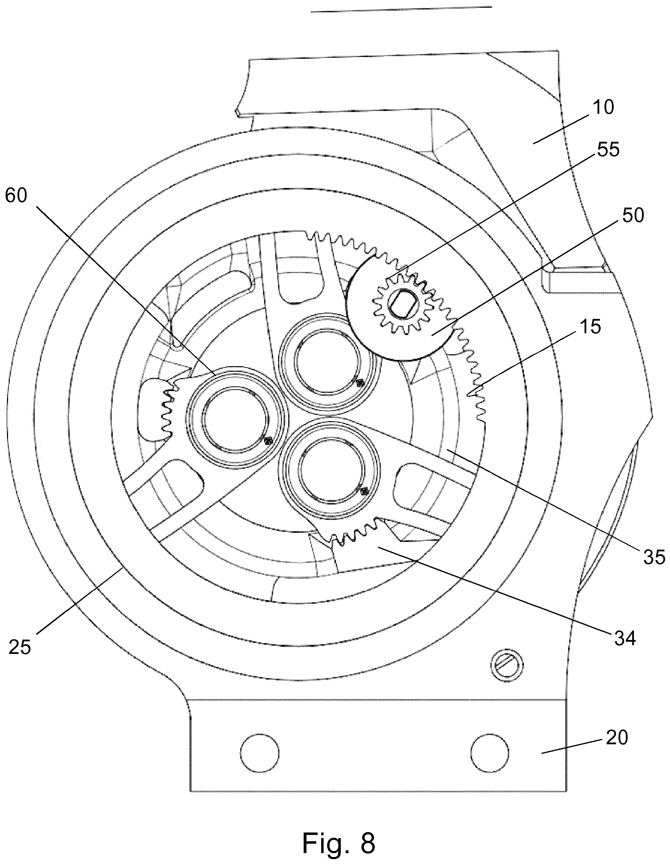

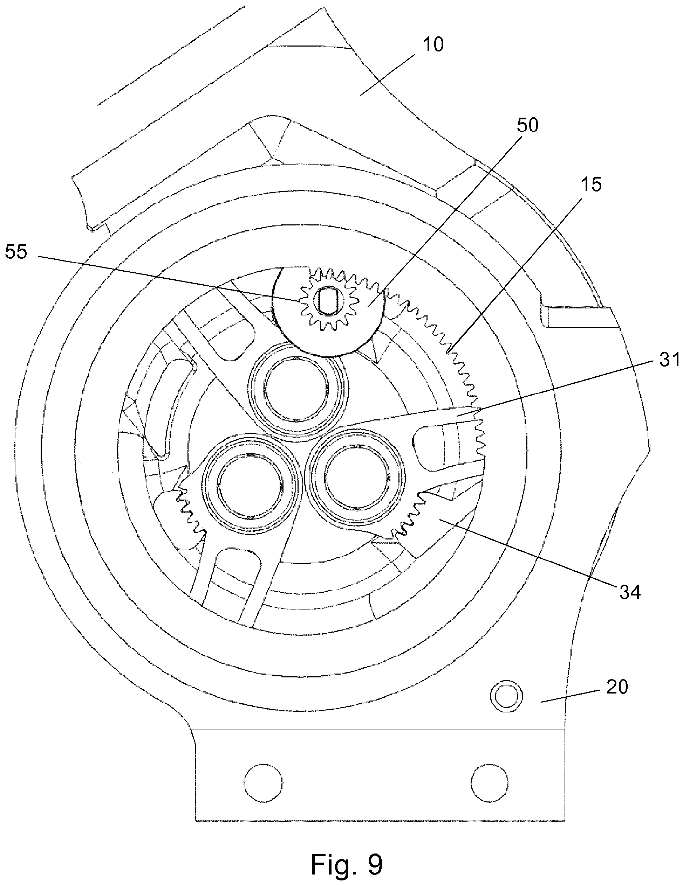

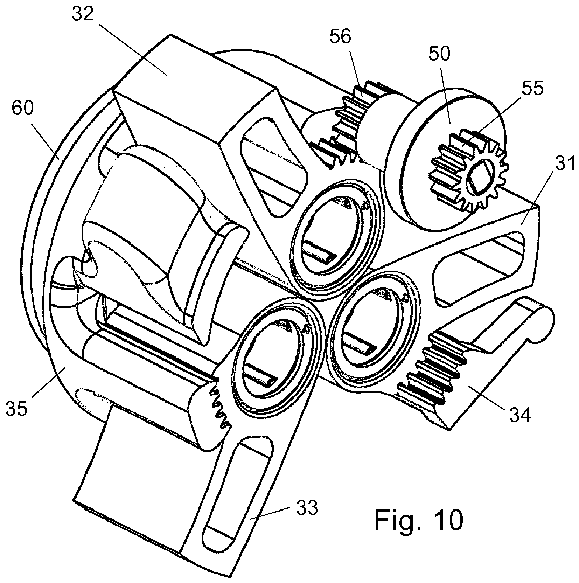

Figur 8 zeigt eine seitliche Darstellung eines Teils des Prothesengelenkes mit dem Oberteil 10, dem Unterteil 20 und dem innerhalb der Bremstrommel angeordneten Aktuator 50, der eine erste Verzahnung 55 aufweist, die auf einer Innenverzahnung 15, die innerhalb der Bremstrommel 25 angeordnet ist, abläuft. Die Bremselemente sind nur teilweise dargestellt, ebenso das Betätigungselement 35 und der Träger 60. Wird das Gelenk verschwenkt, also das Oberteil 10 entgegen dem Uhrzeigersinn verdreht, wie es in der Figur 9 dargestellt ist, läuft das Zahnrad 55 des Aktuators 50 auf der Innenverzahnung 15 ab und versetzt den Aktuator 50 in Drehung, wodurch der Aktuator 50 angetrieben wird. Durch diese Bewegung und den Antrieb des Aktuators 50 durch die Innenverzahnung 15 und das Zahnrad 55 wird eine Kraft auf ein weiteres, innenliegendes Zahnrad 56 ausgeübt, das in der Figur 10 dargestellt ist. Die Verdrehung des ersten Zahnrades 55 führt zu einer Verdrehung des zweite Zahnrades 56 in gleichsinniger Richtung, so dass das Bremselement 32 um den dazugehörigen Zapfen 320 verlagert wird und darüber das Betätigungselement 35 und darüber die anderen Bremselemente 31, 33 in einen deaktivierten Zustand bewegt oder hält. Diese die Bremselemente 31, 32, 33 in den deaktivierten Zustand haltende oder bringende Kraft wird über die gesamte Flexionsbewegung ausgeübt, unabhängig, wie groß die Flexionsbewegung ist. Sobald eine Bewegungsumkehr stattfindet, dreht das erste Zahnrad 55 und damit auch das zweite Zahnrad 56 in die entgegengesetzte Richtung, und die Bremselemente 31, 32, 33 werden aktiv gegen die Bremstrommel gedrückt. Die Verschwenkung erfolgt dann in dieser Darstellung durch eine Bewegung der Bremselemente 32, 32, 33 in dem Uhrzeigersinn um die jeweiligen Lagerzapfen. Der Aktuator 50 arbeitet somit als ein Aktuator, der bewegungsrichtungsabhängig eine Bremseinrichtung deaktiviert. Nach einer Bewegungsrichtungsumkehr von Flexionsrichtung in Extensionsrichtung wird diese deaktivierende Kraftkomponente umgedreht oder aufgehoben und eine Anlage der Bremselemente 31, 32, 33 an die Bremstrommel bewirkt. Dieses Anlegen kann entweder durch den Aktuator 50 allein oder durch ein nicht dargestelltes Rückstellelement, beispielsweise durch Spiralfedern oder Wendelfedern in den Lagerzapfen erreicht werden. Da die Bremselemente 31, 32, 33 unmittelbar an der Bremsfläche der Bremstrommel 25 anliegen, kann dann keine erneute Bewegungsumkehr, also keine erneute Flexion stattfinden. Erst nach Erreichen einer vollständigen Streckung oder einer Streckung soweit, dass der Auslösehebel 42 die Bremselemente über das Betätigungselement 35 und das Bauteil 34 abheben kann, kann eine Flexionsbewegung wieder durchgeführt werden. Der Aktuator 50 kann aus einem Rotationsdämpfer mit dem ersten Zahnrad 55 als Eingang und einem zweiten Zahnrad 56 auf der Rückseite als Ausgang ausgebildet sein. Eine kontinuierliche Drehung an dem Eingangszahnrad 55 verursacht durch die Dämpfung innerhalb des Dämpfungszylinders ein kontinuierliches Moment an dem Ausgangszahnrad 56. Das Moment wechselt seine Richtung bei der erstmaligen Bewegungsumkehr von Flexion in Extension und bringt die Bremselemente 31, 32, 33 wieder in Kontakt mit der Bremsfläche der Bremstrommel 25. Bei einer abermaligen Bewegungsumkehr von Extension zur Flexion verriegelt die Bremseinrichtung 30 das Gelenk.

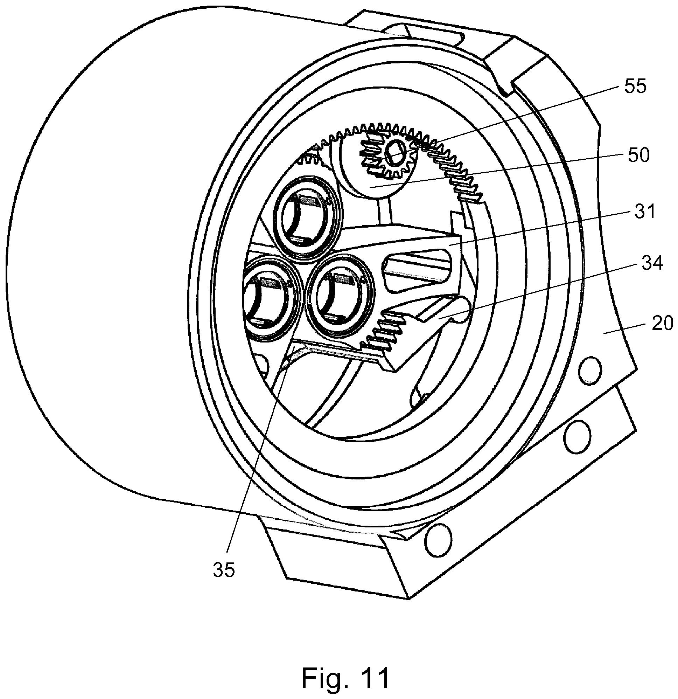

In Figur 11 ist die Verzahnung 15 als Innenverzahnung in einem Ring, der in der Bremstrommel 25 eingesetzt ist, in einer perspektivischen Darstellung gezeigt. Das erste Zahnrad 55 greift in die Innenverzahnung 15 ein und treibt das nur angedeutet zweite Zahnrad 56 zur Verschwenkung des diesem zugeordneten Bremselementes 32 und darüber hinaus auch des Betätigungselementes 35 an.

Alternativ zu einer Ausgestaltung der Bremseinrichtung 30 mit drei Bremselementen 31, 32, 33 kann die Bremseinrichtung 30 auch nur mit einem Bremselement ausgestattet sein, es können auch zwei oder mehr als drei Bremselemente eingesetzt werden.

Alternativ zu einer rein mechanischen Sensierung einer Bewegungsumkehr von Flexion zu Extension kann der Aktuator 50 auch über eine elektronische Sensierung mit entsprechenden Signalen versorgt werden, um über einen elektrischen oder motorisch angetriebenen Aktuator die Bremselemente 31, 32, 33 in einem deaktivierten Zustand zu halten. Sollte nach einer Maximalflexion und einer Bewegungsumkehr zur Extension anschließend eine erneute Flexionsbewegung initiiert werden, bevor der vollständige Streckzustand erreicht ist, indem der Auslösemechanismus 40 wirksam sein kann, wird eine Blockierung des Gelenkes bewirkt.

## Patentansprüche

1. Bremsgelenk mit einem Oberteil (10), einem um eine Schwenkachse (12) schwenkbar daran gelagerten Unterteil (20) und einer Bremseinrichtung (30), die zumindest ein Bremselement (31, 32, 33) aufweist, das über einen Auslösemechanismus (40) aus einem aktivierten, eine Flexionsbewegung zumindest bremsenden Zustand in einen deaktivierten, eine Flexionsbewegung zulassenden Zustand bringbar ist, wobei die Bremseinrichtung (30) eine Extensionsbewegung nicht sperrt und der Bremseinrichtung (30) ein Aktuator (50) zugeordnet ist, der das zumindest eine Bremselement (31,32, 33) während der Flexionsbewegung in dem deaktivierten Zustand hält, **dadurch gekennzeichnet, dass** der Auslösemechanismus (40) einen Auslösehebel (42) aufweist, der mit dem zumindest einen Bremselement (31, 32, 33) gekoppelt ist.

2. Bremsgelenk nach Anspruch 1, **dadurch gekennzeichnet, dass** der Aktuator (50) das zumindest eine Bremselement (31, 32, 33) während der Extensionsbewegung in den aktivierten Zustand bewegt.

3. Bremsgelenk nach Anspruch 1 oder 2, **dadurch gekennzeichnet, dass** dem zumindest einen Bremselement (31, 32, 33) ein Rückstellelement (36) zugeordnet ist, das das zumindest eine Bremselement (31, 32, 33) in Richtung auf den aktivierten Zustand belastet oder bewegt.

4. Bremsgelenk nach einem der voranstehenden Ansprüche, **dadurch gekennzeichnet, dass** das zumindest eine Bremselement (31, 32, 33) in Flexionsrichtung selbstverstärkend und in Extensionsrichtung deaktivierend gelagert ist.

5. Bremsgelenk nach einem der voranstehenden Ansprüche, **dadurch gekennzeichnet, dass** das zumindest eine Bremselement (31, 32, 33) in einer Bremstrommel (25) verschwenkbar an einem Träger (60) gelagert ist.

6. Bremsgelenk nach Anspruch 5, **dadurch gekennzeichnet, dass** ein Betätigungselement (35) verdrehbar zu dem Träger (60) gelagert und mit dem Auslösemechanismus (40) und dem Aktuator (50) gekoppelt ist.

7. Bremsgelenk nach einem der voranstehenden Ansprüche, **dadurch gekennzeichnet, dass** das Unterteil (20) zwei Komponenten (21, 22) aufweist, die um eine Steuerachse (41) verschwenkbar aneinander gelagert sind, um bei einer Auslösebelastung eine Verschwenkbewegung der Unterteilkomponenten (21, 22) zu bewirken, durch die der Auslösemechanismus (40) das zumindest eine Bremselement (31, 32, 33) deaktiviert.

8. Bremsgelenk nach einem der voranstehenden Ansprüche, **dadurch gekennzeichnet, dass** der Aktuator (50) als Rotationsdämpfer ausgebildet ist, der die Flexionsbewegung in eine den deaktivierten Zustand beibehaltende Kraft umwandelt.

9. Bremsgelenk nach einem der voranstehenden Ansprüche, **dadurch gekennzeichnet, dass** der Aktuator (50) über einen Mitnehmer (53) verschwenkbar um die Schwenkachse (12) mit dem Oberteil (10) oder Unterteil (20) gekoppelt ist und die Relativbewegung zwischen Oberteil (10) und Unterteil (20) auf das zumindest ein Bremselement (31, 32, 33) überträgt.

10. Bremsgelenk nach Anspruch 9, **dadurch gekennzeichnet, dass** der Aktuator (50) über eine Verzahnung (15) mit dem Oberteil (10) oder Unterteil (20) gekoppelt ist.

11. Bremsgelenk nach einem der Ansprüche 1 bis 7, **dadurch gekennzeichnet, dass** der Aktuator (50) als sensorgesteuerter Antrieb ausgebildet ist.

12. Bremsgelenk nach einem der voranstehenden Ansprüche, **dadurch gekennzeichnet, dass** der Aktuator (50) bei einer Extensionsbewegung das zumindest eine Bremselement (31, 32, 33) in den aktivierten Zustand bewegt.

13. Bremsgelenk nach einem der voranstehenden Ansprüche, **dadurch gekennzeichnet, dass** es als Orthesenbremsgelenk oder als Prothesenbremsgelenk, insbesondere als Bremskniegelenk ausgebildet ist.

## Claims

1. Braking joint with an upper part (10), a lower part (20) mounted thereon so as to be pivotable about a pivot axis (12) and a braking device (30) which has at least one braking element (31, 32, 33) which can be brought by means of a release mechanism (40) from an activated state at least braking a flexion movement into a deactivated state permitting a flexion movement, wherein the braking device (30) does not block an extension movement and the braking device (30) is associated with an actuator (50) which holds the at least one braking element (31, 32, 33) in the deactivated state during the flexion movement, **characterised in that** the release mechanism (40) has a release lever (42) which is coupled to the at least one braking element (31, 32, 33).

2. Braking joint according to claim 1, **characterised in that** the actuator (50) moves the at least one braking element (31, 32, 33) into the activated state during the extension movement.

3. Braking joint according to claim 1 or 2, **characterised in that** a restoring element (36) is associated with the at least one braking element (31, 32, 33) and loads or moves the at least one braking element (31, 32, 33) in the direction of the activated state.

4. Braking joint according to one of the preceding claims, **characterised in that** the at least one braking element (31, 32, 33) is mounted so as to be self-reinforcing in the flexion direction and deactivating in the extension direction.

5. Braking joint according to one of the preceding claims, **characterized in that** the at least one braking element (31, 32, 33) is pivotably mounted in a brake drum (25) on a carrier (60).

6. Braking joint according to claim 5, **characterised in that** an actuating element (35) is mounted rotatably relative to the carrier (60) and is coupled to the release mechanism (40) and the actuator (50).

7. Braking joint according to one of the preceding claims, **characterized in that** the lower part (20) comprises two components (21, 22) which are pivotally mounted to each other about a control axis (41) in order to cause a pivoting movement of the lower part components (21, 22) upon a release load, by which the release mechanism (40) deactivates the at least one braking element (31, 32, 33).

8. Braking joint according to one of the preceding claims, **characterized in that** the actuator (50) is designed as a rotational damper which converts the flexion movement into a force maintaining the deactivated state.

9. Braking joint according to one of the preceding claims, **characterized in that** the actuator (50) is coupled to the upper part (10) or lower part (20) so as to be pivotable about the pivot axis (12) via a driver (53) and transmits the relative movement between the upper part (10) and lower part (20) to the at least one braking element (31, 32, 33).

10. Braking joint according to claim 9, **characterised in that** the actuator (50) is coupled to the upper part (10) or lower part (20) via a toothing (15).

11. Braking joint according to one of claims 1 to 7, **characterised in that** the actuator (50) is designed as a sensor-controlled drive.

12. Braking joint according to one of the preceding claims, **characterized in that** the actuator (50) moves the at least one braking element (31, 32, 33) into the activated state during an extension movement.

13. Braking joint according to one of the preceding claims, **characterized in that** it is designed as an orthotic braking joint or as a prosthetic braking joint, in particular as a braking knee joint.

## Revendications

1. Articulation avec freinage, comprenant une partie supérieure (10), une partie inférieure (20) montée sur celle-ci de manière à pouvoir pivoter autour d'un axe de pivotement (12), et un dispositif de freinage (30) qui présente au moins un élément de freinage (31, 32, 33) qui, par l'intermédiaire d'un mécanisme de déclenchement (40), passe d'un état activé, freinant au moins un mouvement de flexion, à un état désactivé, autorisant un mouvement de flexion, le dispositif de freinage (30) ne bloquant pas un mouvement d'extension, et un actionneur (50) étant associé au dispositif de freinage (30), lequel maintient ledit au moins un élément de freinage (31, 32, 33) dans l'état désactivé pendant le mouvement de flexion,
**caractérisée en ce que** le mécanisme de déclenchement (40) présente un levier de déclenchement (42) qui est couplé audit au moins un élément de freinage (31, 32, 33).

2. Articulation avec freinage selon la revendication 1,
**caractérisée en ce que** l'actionneur (50) déplace ledit au moins un élément de freinage (31, 32, 33) dans l'état activé pendant le mouvement d'extension.

3. Articulation avec freinage selon la revendication 1 ou 2,
**caractérisée en ce qu'**un élément de rappel (36) est associé audit au moins un élément de freinage (31, 32, 33), lequel sollicite ou déplace ledit au moins un élément de freinage (31, 32, 33) en direction de l'état activé.

4. Articulation avec freinage selon l'une des revendications précédentes, **caractérisée en ce que** ledit au moins un élément de freinage (31, 32, 33) est monté de manière à s'auto-renforcer dans la direction de flexion et à se désactiver dans la direction d'extension.

5. Articulation avec freinage selon l'une des revendications précédentes, **caractérisée en ce que** ledit au moins un élément de freinage (31, 32, 33) est monté sur un support (60) de manière à pouvoir pivoter dans un tambour de freinage (25).

6. Articulation avec freinage selon la revendication 5,
**caractérisée en ce qu'**un élément d'actionnement (35) est monté de manière à pouvoir tourner par rapport au support (60) et est couplé au mécanisme de déclenchement (40) et à l'actionneur (50).

7. Articulation avec freinage selon l'une des revendications précédentes, **caractérisée en ce que** la partie inférieure (20) présente deux composants (21, 22) qui sont montés l'un sur l'autre de manière à pouvoir pivoter autour d'un axe de commande (41), afin de provoquer, lors d'une sollicitation de déclenchement, un mouvement de pivotement des composants de partie inférieure (21, 22) par lequel le mécanisme de déclenchement (40) désactive ledit au moins un élément de freinage (31, 32, 33).

8. Articulation avec freinage selon l'une des revendications précédentes, **caractérisée en ce que** l'actionneur (50) est réalisé sous forme d'amortisseur de rotation qui transforme le mouvement de flexion en une force qui maintient l'état désactivé.

9. Articulation avec freinage selon l'une des revendications précédentes, **caractérisée en ce que** l'actionneur (50) est couplé à la partie supérieure (10) ou à la partie inférieure (20) de manière à pouvoir pivoter autour de l'axe de pivotement (12) par l'intermédiaire d'un entraîneur (53) et transmet le mouvement relatif entre la partie supérieure (10) et la partie inférieure (20) audit au moins un élément de freinage (31, 32, 33).

10. Articulation avec freinage selon la revendication 9,
**caractérisée en ce que** l'actionneur (50) est couplé à la partie supérieure (10) ou à la partie inférieure (20) par l'intermédiaire d'une denture (15).

11. Articulation avec freinage selon l'une des revendications 1 à 7, **caractérisée en ce que** l'actionneur (50) est réalisé sous forme d'entraînement commandé par capteur.

12. Articulation avec freinage selon l'une des revendications précédentes, **caractérisée en ce que** lors d'un mouvement d'extension, l'actionneur (50) déplace ledit au moins un élément de freinage (31, 32, 33) dans l'état activé.

13. Articulation avec freinage selon l'une des revendications précédentes, **caractérisée en ce qu'**elle est réalisée sous forme d'articulation avec freinage pour orthèse ou sous forme d'articulation avec freinage pour prothèse, en particulier sous forme d'articulation de genou avec freinage.
